# EUROPEAN PATENT SPECIFICATION

(11) **EP 2 919 878 B1**
(45) Date of publication and mention of the grant of the patent: **04.03.2020**
(21) Application number: 13798601.4
(22) Date of filing: 14.11.2013
(51) Int. Cl.: C07C 209/86, B01D 17/12

(54) **USE OF A DEVICE COMPRISING A TRANSPARENT SAPPHIRE OR MICA DISK FOR SEPARATING IMMISCIBLE PHASES IN A PROCESS FOR THE PREPARATION OF DI-AND POLYAMINES OF THE DIPHENYL METHANE SERIES**
VERWENDUNG EINER VORRICHTUNG MIT EINER DURCHSICHTIGEN SAPHIR- ODER GLIMMERSCHEIBE ZUR TRENNUNG NICHT MISCHBARER PHASEN IN EINEM VERFAHREN ZUR HERSTELLUNG VON DI- UND POLYAMINEN DER DIPHENYLMETHAN-REIHE
UTILISATION D'UN DISPOSITIF COMPRENANT UN DISQUE TRANSPARENT EN SAPHIR OU EN MICA POUR SÉPARER DES PHASES IMMISCIBLES DANS UN PROCÉDÉ POUR LA PRÉPARATION DE DI-ET POLYAMINES DE LA SÉRIE DE DIPHENYL METHANE

(30) Priority: 15.11.2012 US 201213677420
(43) Date of publication of application: 23.09.2015
(73) Proprietor: Covestro Deutschland AG, 51373 Leverkusen (DE)
(72) Inventor: TEMME, Bodo, 41542 Dormagen (DE); FRUHEN, Bernd, 47809 Krefeld (DE); KNAUF, Thomas, 41542 Dormagen (DE); WERSHOFEN, Stefan, 41065 Mönchengladbach (DE); ADAMSON, Richard, 42799 Leichlingen (DE); PAURA, Wolfgang, 25348 Glückstadt (DE); DADD, Susan, League City, Texas 77573 (US); ESSER, Ralf, Houston, Texas 77058 (US)
(74) Representative: Levpat
(86) International application number: PCT/EP2013/073860
(87) International publication number: WO 2014/076197

(56) References cited:
- WO-A2-03/072215
- DE-A1- 2 549 890
- DE-U1- 8 810 807
- US-B1- 6 174 956
- US-B1- 6 566 410

## Description

The present invention relates to the use of a device for separating two immiscible phases (phase separation device) composed of at least one vessel for receiving the at least two phases, at least one pipe for supplying a fluid to the vessel, at least two pipes for discharging fluids from the vessel, and at least one means for observing the separation operation that includes a transparent disk, in a process for the preparation of di- and polyamines of the diphenylmethane series. At least the side of the transparent disk that faces the phases to be separated is made of sapphire (sapphire glass) or mica (mica disk).

Many chemical processes comprise steps in which two or more immiscible phases are separated from one another (for example an organic phase containing the desired product is separated from the water of reaction that forms), as well as steps in which one phase is extracted with another, immiscible phase (for example an organic phase containing the desired product is washed with water). The apparatuses, phase separation or extraction vessels, used for this purpose have been known in principle from the prior art for a long time. It is conventional to equip such apparatuses with sight glasses, which allow visual monitoring of the phase separation or extraction operation. Such visual monitoring is important because it can happen that phase separation operations, for example, are disrupted by the formation of stable emulsions. In the preparation of di- and polyamines of the diphenylmethane series, in connection with the separation of the organic, product-containing phase from the aqueous phase, the formation of a third phase after the neutralisation of the crude product has been reported, which third phase impedes the phase separation or even makes it impossible (see EP 1 652 835 A1). When there is the possibility of visually observing the phase separation or extraction operation, such undesirable effects can be detected at an early stage, when they can still be counteracted, for example, by adjusting certain process parameters. The sight glasses required therefor are conventionally manufactured from quartz glass or borosilicate glass. Quartz glass and borosilicate glass have the disadvantage, however, of undergoing damage when used continuously, in particular when alkaline media are present. Alkaline corrosion, erosion and, in the worst case, leakages can occur. For some special applications, which go beyond phase separation or extraction, such as the evaporation of concentrated alkali solutions in dye production, sight glasses that are protected by a polytetrafluoroethylene layer, which is intended to prevent corrosion of the glass, are also used (see utility model specification CN 201578869 U). However, it must be assumed that the optical transparency suffers as a result of the polytetrafluoroethylene layer. The use of this system in phase separation or extraction vessels is therefore unsatisfactory.

DE 88 10 807 U1 is directed to a device for extraction of a solid with an extracting agent that is liquid under the conditions of extraction. The device is described as an autoclave. This device has, in the embodiment of Figure 1, no discharge pipes at all, which means that the material to be extracted and the liquid extracting agent can only be removed when the device is opened after completion of the extraction process. This is, therefore, clearly a device for discontinuous extraction. In the embodiment of Figure 2, only the liquid can be removed via a single line 5. The material to be extracted can only be removed upon opening the apparatus. Thus, also in this embodiment, DE 88 10 807 U1 only discloses a device for discontinuous extraction.

US 6,174,956 B1 relates to a polyurethane polymer and a method of preparing the same. The process requires the combination of a soluble polyol with a di- or polyisocyanate under densified fluid conditions. The process to prepare a waterborne polyurethane involves the utilization of a polyol containing ionic or latent ionic groups which must undergo pre-neutralization before combining with the di- or polyisocyanate under densified fluid conditions. The resulting polyurethane is substantially free of an organic solvent. In the experimental section, solubility tests of various monomers (for example methylene di-p-phenyl diisocyanate) are reported. The solubility was tested qualitatively using a stainless steel, high pressure view cell equipped with two sapphire windows.

US 6,566,410 B1 relates to a method of demulsifying a water-in-oil emulsion comprising an oil phase comprising asphaltenes and an aqueous phase. The method comprises contacting a carbon dioxide containing fluid with the emulsion such that the carbon dioxide containing fluid enters the oil phase of the emulsion. The asphaltenes precipitate out of the emulsion and the emulsion destabilizes. Demulsification experiments are described that were conducted in a cylindrical shaped stainless steel high-pressure cell supplied with two sapphire windows, the cell having an inlet gas valve and an outlet gas valve connected to a pressure rupture disk.

WO 03/072215 A2 relates to a method of dissolving a petroleum-based substance, comprising introducing the petroleum-based substance to a reaction vessel, contacting the petroleum-based substance with a carbon dioxide miscible solvent introduced to the reaction vessel, dissolving at least a portion of the petroleum-based substance in the carbon dioxide miscible solvent contacting the petroleum-based substance, removing a product from the reaction vessel, wherein the product comprises at least a portion of the petroleum-based substance dissolved in at least a portion of the solvent, and contacting the product with carbon dioxide such that the petroleum-based substance is precipitated from the product. In the experimental section, a protocol for single-batch extraction of tar sand samples using CO₂ is disclosed. Tar sand samples are placed in high-pressure reactors that are subsequently heated and pressurized with CO₂. After 1 hour, CO₂ and the extracted bitumen are conveyed under pressure to another reactor supplied with sapphire windows (view-cell). After cooling the extract to ambient temperature, CO₂ is slowly discharged from the system. The system is thereafter opened, the extracted bitumen is collected quantitatively by dissolving in methylene chloride, and the solvent is evaporated in a vacuum oven.

DE 25 49 890 A1 relates to an improved process for the production of polyamines of the diphenylmethane series by the condensation of aniline and formaldehyde in the presence of an acid catalyst. More particularly, this invention relates to the separation of the polyamine, salt and water reaction mixture, which is generated by neutralizing the acidic mixture with sodium hydroxide when hydrochloric acid is used as the catalyst, into first organic and aqueous phases, followed by the treatment of the first organic phase with water at temperatures above 70 °C, after which this mixture is separated into second organic and aqueous phases and the second organic phase is fractionated to recover polyamine product. Means for observing any of the phase separations are not disclosed.

There was, therefore, a need for a device to be used in a process for the preparation of di- and polyamines of the diphenylmethane series which allows phase separation operations to be observed without having the disadvantages described above.

Having regard to this need, the present invention provides a use of a device for separating two immiscible phases composed of at least one vessel for receiving the at least two phases, at least one pipe for supplying a fluid to the vessel, at least two pipes, for discharging fluids from the vessel, and at least one means for observing the separation operation that includes a transparent disk with at least the side of the transparent disk that faces the phases to be separated being made of sapphire or mica, preferably of sapphire,
in a process for the preparation of di- and polyamines of the diphenylmethane series comprising:
reacting aniline with formaldehyde in the presence of an acidic catalyst;
neutralizing the reaction mixture with a base followed by separating the organic phase from the aqueous phase;
working-up the organic phase that remains after separation of the aqueous phase;
freeing the organic phase from excess aniline by distillation, extraction or crystallization;
wherein the device is used at least for separating, after neutralization of the reaction mixture, the organic phase from the aqueous phase.

*Fluids* within the scope of the present invention denote liquid streams, which may, however, also contain gaseous or solid components. A fluid supplied to the device by way of the at least one pipe is at least the two-phase liquid reaction mixture obtained in the neutralization of the reaction mixture, which is separated in the device into an organic phase (one of the fluids to be discharged from the device) and an aqueous phase (likewise one of the fluids to be discharged from the device).

Within the context of the present invention, the term *"sapphire"* is understood as meaning materials that consist substantially (to the extent of at least 90.0 % by mass, preferably at least 95.0 % by mass, particularly preferably at least 99.0 % by mass, most particularly preferably at least 99.9 % by mass, in each case based on the total mass of the sapphire material) of aluminium oxide and have a corundum structure. The production of sapphires is sufficiently well known from the prior art. Sapphire crystals having large diameters can be produced, for example, by means of the Nacken-Kyropoulos process, the Czochralski or crystal pulling process, the Stepanov process / EFG technique, the Tammann-Stöber process, the heat-exchanger process and the Bridgman process; see, for example, the internet page "http://www.finepowder.de/Aluminiumoxid_fuer_Saphir.html". Sapphire glasses are used in the prior art in vacuum technology and spectroscopy as well as in high-quality watches. A use of sapphire glasses as provided according to the invention has not hitherto been known from the prior art. The transparent sapphires to be used according to the invention are also referred to as *sapphire glasses.* The terms are used synonymously within the context of this invention.

Within the context of the present invention *mica* is understood as meaning a material that consists substantially (to the extent of at least 90.0 % by mass, preferably at least 95.0 % by mass, particularly preferably at least 99.0 % by mass, most particularly preferably at least 99.9 % by mass, in each case based on the total mass of mica material) of sheet silicates of the general composition **DG_{2.3}[T₄O₁₀]X₂,** wherein:
**D** denotes 12-fold coordinated cations selected from the group consisting of potassium, sodium, calcium, barium, rubidium, caesium and ammonium cations;
**G** denotes 6-fold coordinated cations selected from the group consisting of lithium, magnesium, iron(II and III), manganese(II), zinc, aluminium, chromium, vanadium and titanium cations;
**T** denotes 4-fold coordinated cations selected from the group consisting of silicon, aluminium, iron(III), boron and beryllium cations;
**X** denotes an anion selected from the group consisting of OH⁻, F⁻, Cl⁻, O²⁻ and S²⁻.

The production of mica disks is known from the prior art. Mica is used industrially, for example, in automotive paints, cosmetics, as an electrical insulator, as an insulating disk or as a viewing window in ovens (high temperature stability). A use of mica as provided according to the invention has not hitherto been known from the prior art. The transparent micas to be used according to the invention are also referred to as *mica disks.* The terms are used synonymously within the context of this invention.

Embodiments of the invention are described below. Different embodiments can be combined with one another as desired, unless the context suggests otherwise.

In one embodiment, the transparent disk consists wholly of sapphire glass or of mica, preferably of sapphire.

In a further embodiment, only the side of the transparent disk that faces the phases to be separated or extracted consists of sapphire or mica, preferably of sapphire. In this embodiment, the arrangement comprising a transparent disk (called sight glass hereinbelow) is accordingly a composite of sapphire or mica (the side that is exposed to the phases to be separated or extracted) and another transparent material (the side that is remote from the phases to be separated or extracted), preferably borosilicate glass or quartz glass. The construction of the sight glass in this variant can take place by means of separate sapphire glass disks / mica disks and borosilicate glass disks or sapphire glass disks/mica disks and quartz glass disks, or a sapphire glass layer / mica layer can be applied to borosilicate glass or quartz glass. Bonding between sapphire or mica and the second transparent material to form a composite can take place by means of methods known to the person skilled in the art for producing a composite of different materials. In the simplest case, the two layers are pressed together and joined together by means of a frame. Adhesive bonding of the disks is also conceivable.

Apart from the sight glass for observing the separation operation or the extraction operation, the phase separation or extraction device according to the invention corresponds to corresponding devices of the prior art, as are described, for example, in Mass-Transfer Operations, Third Edition, International Edition 1981, McGraw-Hill Book Co, p. 477 to 541, or Ullmann's Encyclopedia of Industrial Chemistry (Vol. 21, Liquid-Liquid Extraction, E. Müller et al., pages 272-274, 2012 Wiley-VCH Verlag GmbH & Co. KGaA, Weinheim, DOI: 10.1002/14356007.b03_06.pub2) or in Kirk-Othmer Encyclopedia of Chemical Technology (see "http://onlinelibrary.wiley.com/book/10.1002/0471238961", Published Online: 15 June 2007, pages 22-23) (mixer-settler cascade or settling vessel).

Apart from the material of the transparent disk, a sight glass to be used according to the invention likewise corresponds to the prior art which is conventional in chemical process technology for separation or extraction vessels. The processing of sapphire and mica is sufficiently well known from the prior art. Depending upon the task to be carried out, a separation or extraction vessel can have one or more sight glasses which are so arranged to permit sufficiently close observation of the separation or extraction operation. In order to prevent electrostatic charging, the sight glass can be coated with indium tin oxide.

The invention provides a use of the device , in a process for the preparation of di- and polyamines of the diphenylmethane series. Within the context of the present invention, the expression *"diamines of the diphenylmethane series"* denotes the various isomers of so-called monomeric diaminodiphenylmethane (MDA hereinbelow), H₂N-C₆H₄-CH₂-C₆H₄-NH₂, while the expression *"polyamines of the diphenylmethane series"* (PMDA hereinbelow) denotes, in addition to the mentioned diamines of the diphenylmethane series, also higher-nuclear (i.e. tri- and/or poly-nuclear) compounds having three or more amino groups. The same is true of the corresponding isocyanates.

The preparation of MDA and PMDA with the main component MDA by reaction of aniline with formaldehyde in the presence of acidic catalysts is generally known. The di- and polyamine mixtures are used predominantly for the preparation of the corresponding di- and polyisocyanates (MDI and PMDI). Examples of continuous or semi-batchwise processes for the preparation of di- and poly-amines of the diphenylmethane series (MDA and PMDA) are disclosed in US 5,286,760, EP-A-0 451 442 and WO-A-99/40059.

For working up of the acidic reaction mixture, the reaction mixture is neutralized with a base according to the prior art. According to the prior art, neutralization is conventionally carried out at temperatures of, for example, from 90°C to 100°C without the addition of further substances (see H. J. Twitchett, Chem. Soc. Rev. 3(2), 223 (1974)). However, it can also be carried out at a different temperature level in order, for example, to accelerate the degradation of disruptive secondary products. Suitable bases are, for example, the hydroxides of the alkali and alkaline earth elements, preferably in the form of an aqueous solution. Hydroxides of the alkali elements are preferably suitable, and sodium hydroxide is particularly preferably used. Most particular preference is given to the use of sodium hydroxide solution, the concentration of sodium hydroxide being from 10 to 50 wt.%, preferably from 25 to 50 wt.%.

The neutralization is generally not carried out exactly to the neutral point; rather, an excess of base is used, so that the resulting aqueous phase is alkaline. Further details of the neutralisation can be found in EP 1 616 890 A1, in particular paragraphs [0038] to [0039], to which reference is hereby made.

Following the neutralization, the organic phase is separated from the aqueous phase in a separation vessel. The product-containing organic phase that remains after separation of the aqueous phase is subjected to further working-up steps (e.g. washing, see DE-A 25 49 890) and then freed of excess aniline and other substances present in the mixture (e.g. further solvents) by suitable processes such as, for example, distillation, extraction or crystallization. The above-described device is excellently suitable for the above-mentioned phase separation and extraction (washing) steps and is used at least for the phase separation following the neutralization. Accordingly, the present invention relates to the use of the device in the separation of an aqueous phase and an organic phase containing di- and polyamines of the diphenylmethane series. The invention further provides the use of the device according to the invention in the separation of an aqueous phase and an organic phase containing di- and polyamines of the diphenylmethane series, in which the aqueous phase is alkaline and in particular has a pH value of from 8.0 to 14.

The use of the device in the preparation of di- and polyamines of the diphenylmethane series has many advantages:
i) There is virtually no corrosion of the sight glass at the high temperatures in the neutralisation and washing vessel and by the alkaline medium. The frequency of damage is reduced drastically.
ii) The product quality does not suffer, because sight glasses of sapphire and mica remain transparent and do not, like conventional glass, become milky, with the result that, when conventional glass is used, a meaningful assessment of the phase separation operations in the vessels is no longer possible correctly after only a short time.
iii) Energy costs are saved because frequent starting and stopping for repair or replacement of the sight glass is avoided.
iv) There are no safety problems because of product emerging through leakages in the sight glass.
v) Maintenance costs are saved because there are no production downtimes and no repair costs are incurred.

The di- and polyamines of the diphenylmethane series so obtained can be reacted with phosgene according to known methods to give the corresponding di- and polyisocyanates of the diphenylmethane series.

### EXAMPLES

### Construction and fitting of the sight glasses:

Mounting of the sight glass is carried out in accordance with DIN 28120 (Circular sight glasses with case in main power connection). As the gasket there is used a graphite gasket with a steel insert of 1.4401 (gasket code letter NK).

The borosilicate sight glasses are produced according to DIN 7080. The sight glasses with sapphire or mica were produced, apart from the material, according to the same specification.

### General specification for working up crude di- and polyamines of the diphenylmethane series

32% sodium hydroxide solution is added in a molar ratio of 1.1 : 1 (sodium hydroxide solution to HCl) to an HCl-acidic reaction mixture containing *inter alia* the desired di- and polyamines of the diphenylmethane series and excess aniline and water, and the mixture is reacted to completion in a stirred neutralisation vessel. The temperature is from 80°C to 130°C and the absolute pressure is from 0.7 to 2.0 bar.

The resulting mixture is then separated in a neutralisation separator, which is equipped with a sight glass, into an aqueous, lower phase which is fed to the waste water collection vessel. This water has a pH value of about 13, a NaCl content of about 21 wt.% and a NaOH concentration of about 2 wt.%. The organic, upper phase is fed to washing. In the stirred washing vessel, the organic phase is washed with condensed water vapour. After the wash water has been separated off in the wash water separator, which is equipped with a sight glass, the moist mixture of MDA and PMDA is pumped into a collecting vessel. The wash water which has been separated off, which has a pH value of about 11, a NaCl content of about 0.2 wt.% and a NaOH concentration of about 0.8 wt.%, is likewise transferred to the waste water collecting vessel. The water from the waste water collecting vessel, which consists of the water of the neutralisation, the washing and other water streams from the reaction and distillation and which has a pH value of about 13, a NaCl content of about 7 wt.% and a NaOH concentration of about 0.8 wt.%, is extracted with fresh aniline in the waste water extraction.

### Example 1 (comparison)

Use of a conventional sight glass of borosilicate glass in all apparatuses equipped with a sight glass. Because leakages occur at random, there are unplanned stoppages in production.

### Example 2 (according to the invention)

Use of a borosilicate sight glass protected on the product side with a mica disk in all apparatuses equipped with a sight glass.

### Example 3 (according to the invention)

Use of a sapphire sight glass in all apparatuses equipped with a sight glass. There are no leakages. The sapphire glass does not become scratched either.

### Example 4 (according to the invention)

Use of a two-layer sight glass of sapphire glass (product side) and borosilicate glass (on the side that is remote from the product) in all apparatuses equipped with a sight glass.

The table below summarises the results:

**Table: Material, lifetime, damage**

| | **Neutralisation separator** | | **Washing** | | **Waste water extraction** | |
|---|---|---|---|---|---|---|
| **Example** | **Lifetime (months)** | **Damage** | **Lifetime (months)** | **Damage** | **Lifetime (months)** | **Damage** |
| **1 (comparison)** | 6 | pitted | 12- 15 | pitted | 12 | pitted |
| **2 (according to the invention, mica/ borosilicate)** | >24 | none | >24 | none | >24 | none |
| **3 (according to the invention, solid sapphire)** | >36 | none | >36 | none | >36 | none |
| **4 (according to the invention, sapphire/ borosilicate)** | >24 | none | >24 | none | >24 | none |

## Claims

1. Use of a device for separating at least two immiscible phases in a process for the preparation of di- and polyamines of the diphenylmethane series,
the process comprising:
reacting aniline with formaldehyde in the presence of an acidic catalyst;
neutralizing the reaction mixture with a base followed by separating the organic phase from the aqueous phase;
working-up the organic phase that remains after separation of the aqueous phase;
freeing the organic phase from excess aniline by distillation, extraction or crystallization;
wherein the device is used at least for separating, after neutralization of the reaction mixture, the organic phase from the aqueous phase;
the device comprising:
a) at least one vessel for receiving the phases to be separated,
b) at least one pipe for supplying a fluid to the vessel,
c) at least two pipes for discharging fluids from the vessel, and
d) at least one means for observing the vessel's contents during phase separation comprising:
(i) a transparent disk composed of sapphire or mica on the disk's side that faces the phases to be separated.

2. The use according to Claim 1, in which the transparent disk is composed completely of sapphire or mica.

3. The use according to Claim 1, in which only the side of the transparent disk that faces the phases to be separated is composed of sapphire or mica.

4. The use according to Claim 3 in which the disk of the observation means is a composite of sapphire or mica and another transparent material selected from the group consisting of borosilicate glass and quartz glass.

5. The use according to any one of Claims 1 to 4 in which at least the side of the transparent disk that faces the phases to be separated consists of sapphire.

6. The use according to any one of Claims 1 to 5, in which the neutralization is carried out with an excess of base so that the aqueous phase is alkaline.

7. The use according to Claim 6, in which the pH value of the aqueous phase is from 8.0 to 14.

8. The use according to any one of Claims 1 to 7, in which the working-up of the organic phase comprises washing.

9. The use according to Claim 8, in which the device is also used for the washing step.

## Patentansprüche

1. Verwendung einer Vorrichtung zum Trennen von mindestens zwei nicht mischbaren Phasen in einem Verfahren zur Herstellung von Di- und Polyaminen der Diphenylmethan-Reihe,
wobei das Verfahren Folgendes umfasst:
Umsetzen von Anilin mit Formaldehyd in Gegenwart eines sauren Katalysators;
Neutralisieren der Reaktionsmischung mit einer Base und anschließendes Abtrennen der organischen Phase von der wässrigen Phase;
Aufarbeiten der nach Abtrennung der wässrigen Phase verbleibenden organischen Phase;
Befreien der organischen Phase von überschüssigem Anilin durch Destillation, Extraktion oder Kristallisation;
wobei die Vorrichtung mindestens für das Abtrennen der organischen Phase von der wässrigen Phase nach der Neutralisation der Reaktionsmischung verwendet wird; wobei die Vorrichtung Folgendes umfasst:
a) mindestens einen Behälter zur Aufnahme der zu trennenden Phasen,
b) mindestens eine Leitung zum Zuführen eines Fluids zu dem Behälter,
c) mindestens zwei Leitungen zum Ablassen von Fluiden aus dem Behälter und
d) mindestens ein Mittel zur Beobachtung des Inhalts des Behälters während der Phasentrennung, umfassend:
(i) eine durchsichtige Scheibe, wobei die den zu trennenden Phasen zugewandte Seite der Scheibe aus Saphir oder Glimmer besteht.

2. Verwendung nach Anspruch 1, wobei die durchsichtige Scheibe vollständig aus Saphir oder Glimmer besteht.

3. Verwendung nach Anspruch 1, wobei lediglich die den zu trennenden Phasen zugewandte Seite der Scheibe aus Saphir oder Glimmer besteht.

4. Verwendung nach Anspruch 3, wobei es sich bei der Scheibe des Beobachtungsmittels um einen Verbund aus Saphir oder Glimmer und einem weiteren durchsichtigen Werkstoff aus der Gruppe bestehend aus Borosilicatglas und Quarzglas handelt.

5. Verwendung nach einem der Ansprüche 1 bis 4, wobei mindestens die den zu trennenden Phasen zugewandte Seite der Scheibe aus Saphir besteht.

6. Verwendung nach einem der Ansprüche 1 bis 5, wobei die Neutralisation mit einem Überschuss an Base durchgeführt wird, sodass die wässrige Phase alkalisch ist.

7. Verwendung nach Anspruch 6, wobei die wässrige Phase einen pH-Wert von 8,0 bis 14 aufweist.

8. Verwendung nach einem der Ansprüche 1 bis 7, wobei das Aufarbeiten der organischen Phase einen Waschschritt umfasst.

9. Verwendung nach Anspruch 8, wobei die Vorrichtung auch für den Waschschritt verwendet wird.

## Revendications

1. Utilisation d'un dispositif pour séparer au moins deux phases non miscibles dans un procédé de préparation de di- et polyamines de la série du diphénylméthane,
le procédé comprenant :
la réaction d'aniline avec du formaldéhyde en présence d'un catalyseur acide ;
la neutralisation du mélange réactionnel avec une base, puis la séparation de la phase organique de la phase aqueuse ;
le traitement final de la phase organique qui reste après séparation de la phase aqueuse ;
la libération de la phase organique de l'aniline en excès par distillation, extraction ou cristallisation ;
dans laquelle le dispositif est utilisé au moins pour séparer, après neutralisation du mélange réactionnel, la phase organique de la phase aqueuse ;
le dispositif comprenant :
a) au moins une cuve pour recevoir les phases à séparer,
b) au moins un tuyau pour fournir un fluide à la cuve,
c) moins deux tuyaux pour évacuer des fluides de la cuve, et
d) au moins un moyen pour observer le contenu de la cuve pendant la séparation de phases comprenant :
(i) un disque transparent composé de saphir ou de mica sur le côté du disque qui fait face aux phases à séparer.

2. Utilisation selon la revendication 1, dans laquelle le disque transparent est entièrement composé de saphir ou de mica.

3. Utilisation selon la revendication 1, dans laquelle seul le côté du disque transparent qui fait face aux phases à séparer est composé de saphir ou de mica.

4. Utilisation selon la revendication 3 dans laquelle le disque du moyen d'observation est un composite de saphir ou de mica et d'un autre matériau transparent choisi dans le groupe constitué par le verre de borosilicate et le verre de quartz.

5. Utilisation selon l'une quelconque des revendications 1 à 4 dans laquelle au moins le côté du disque transparent qui fait face aux phases à séparer consiste en du saphir.

6. Utilisation selon l'une quelconque des revendications 1 à 5, dans laquelle la neutralisation est réalisée avec un excès de base, de sorte que la phase aqueuse est alcaline.

7. Utilisation selon la revendication 6, dans laquelle la valeur de pH de la phase aqueuse est de 8,0 à 14.

8. Utilisation selon l'une quelconque des revendications 1 à 7, dans laquelle le traitement final de la phase organique comprend un lavage.

9. Utilisation selon la revendication 8, dans laquelle le dispositif est également utilisé pour l'étape de lavage.
